# EUROPEAN PATENT SPECIFICATION

(11) **EP 1 478 380 B1**
(45) Date of publication and mention of the grant of the patent: **02.08.2006**
(21) Application number: 03712535.8
(22) Date of filing: 26.02.2003
(51) Int. Cl.: A61K 38/00

(54) **USE OF TYROSINE KINASE INHIBITORS FOR TREATING CNS DISORDERS**
VERWENDUNG VON TYROSINE-KINASE INHIBITOREN ZUR BEHANDLUNG VON CNS KRANKHEITEN
UTILISATION D'INHIBITEURS DE TYROSINE KINASE POUR LE TRAITEMENT DE TROUBLES DU CNC

(30) Priority: 27.02.2002 US 359652 P
(43) Date of publication of application: 24.11.2004
(73) Proprietor: AB Science, 75008 Paris (FR)
(72) Inventor: MOUSSY, Alain, F-75006 Paris (FR); KINET, Jean-Pierre, Lexington, MA 02421 (US)
(74) Representative: Warcoin, Jacques
(86) International application number: PCT/IB2003/001425
(87) International publication number: WO 2003/072090

(56) References cited:
- EP-A1- 0 564 409
- WO-A-99/61028
- US-A- 5 952 374

### SEQUENCE LISTING

<110> AB Science
<120> Use of tyrosine kinase inhibitors for treating CNS disorders
<130> D20044 NT
<150> US 60/359,652
   <151> 2002-02-27
<160> 5
<170> PatentIn Ver. 2.1
<210> 1
   <211> 976
   <212> PRT
   <213> Homo sapiens
<220>
   <223> Human c-kit
<400> 1
<210> 2
   <211> 30
   <212> DNA
   <213> Homo sapiens
<220>
   <223> Primer
<400> 2
   aagaagagat ggtacctcga ggggtgaccc 30
<210> 3
   <211> 33
   <212> DNA
   <213> Homo sapiens
<220>
   <223> Primer
<400> 3
   ctgcttcgcg gccgcgttaa ctcttctcaa cca 33
<210> 4
   <211> 20
   <212> DNA
   <213> Homo sapiens
<220>
   <223> Primer
<400> 4
   agctcgttta gtgaaccgtc 20
<210> 5
   <211> 20
   <212> DNA
   <213> Homo sapiens
<220>
   <223> Primer
<400> 5
   gtcagacaaa atgatgcaac 20

## Description

The present invention relates to a method for treating CNS disorders, more particularly selected from the group consisting of depression, schizophrenia, anxiety, migraine, memory loss, pain and neurodegenerative diseases, comprising administering a compound capable of depleting mast cells to a human in need of such treatment. Such compounds can be chosen from tyrosine kinase inhibitors and more particularly non-toxic, selective and potent c-kit inhibitors. Preferably, said inhibitor is unable to promote death of IL-3 dependent cells cultured in presence of IL-3.

Neurons propagate a signal in the form of an action potential along its axon to other neurons or to effector cells. Many positive or negative signals are exchanged between neurons and are integrated to produce meaningful firing patterns. The communication between two neurons is based on the action of numerous neurotransmitters on specific receptors located at the synapses. A disruption in the regulation of neurotransmission is responsible for neurologic and psychiatric diseases. Furthermore, the activity of neurotransmitters on their respective receptor is normally time limited so that receptors can respond repeatedly to the next waves of stimuli. In this regard, different mechanisms abolish the action of neurotransmitters : they can be pumped back into the presynaptic nerve terminals by active processes (reuptake), they can be destroyed by enzymes, or they simply diffuse into the surrounding area.

According to The Merck Manual of Diagnosis and Therapy, Section 14, Neurologic Disorders, changes in neurotransmitter synthesis, storage, release, or degradation or changes in the number and affinity of receptors can affect neurotransmission and cause clinical disorders.

Among neurotransmitters, we can cite glutamate and aspartate, which are the major excitatory neurotransmitters, whereas aminobutyric acid (GABA) is the major inhibitory neurotransmitter in the brain. But, the first theory about depression concerned the noradrenergic system (NS) (Shildkraut J. et al. 1965, Am J. Psychiat. 122, 509-522). At that time, it was observed that tricyclic compounds (ADT) and monoamine-oxidase inhibitors modified the level of noradrenaline. Later on, in 1978, Sulser F. et al., Biochem Pharmacol. 27, 257-261 showed that these antidepressants lead to a decrease in the number of post-synaptic β-adrenergic receptors. Therefore, it was thought that depression was due to the deregulation of the noradrenergic pre-synaptic stimuli as well as the post-synaptic receptors (Siever LJ. et al. (1985), Am. J. Psychiat. 142, 1017-1031). In 1986, Rasmussen et al., Brain Res. 385, 395-400 demonstrated the presence of serotonin (5-hydroxytriptamin, 5-HT) receptors in NA neurones. Treatments with ADT was shown to provoke also a down-regulation of the 5-HT2 receptors in Sugrue M.F. et al, 1981, Pharmacol. Ther. 13, 219-247. As a consequence, it appears that the NA and 5-HT systems play a crucial role in the regulation of mood and behaviour.

In the nineties, research has focused on the finding of specific serotonin re-uptake inhibitors (SSRI), such as fluoxetin, parxetin or sertralin (Pinder R.M. et al., 1993, Med. Res. Rev. 13, 259-325). Serotonin (5-hydroxytryptamine, or 5-HT) levels are controlled by the uptake of tryptophan and intraneuronal monoamine oxidase activity.

In the meantime, a decrease in the level of HVA, the main catabolic of dopamin (DA), was observed in depressed patients (Kapur S. et al., 1992, Biol. Psychiat. 32, 1-17). GABA was also shown to be involved in the physiopathology of depression since (i) unipolar patients display decreased level of GABA, (ii) some antidepressants induce the release of GABA in vivo and (iii) agonists of GABA receptors have antidepressant effects (Lloyd K.G. et al., 1989, Prog. Neuro-Psychopharmacol. Biol. Psychiat. 13, 341-351).

More recently, it has been reported that other factors may be involved in CNS disorders. For example, it has been observed from 30 to 70 % of patients afflicted with melancholia have high level of plasmatic cortisol and escape to the test with dexamethasone described in Caroll B.J. et al. (1981), Arch. Gen. Psychiat. 38, 15.
In addition, corticosteroïds modify (i) the expression of serotoninergic receptors and (ii) the activity of tryptophan hydroxylase, which is the key enzyme in the synthesis of 5-HT (Biegon A., 1990, Ann. NY Acad. Sci. 600,427-431).
Regarding post-partum or post-menopause depression, repeated administration of oestrogene induces a down-regulation of dopaminergic D2 receptors (Munemura M. et al., 1989, Endocrinology 124, 346-355 and Roy E.J. et al., 1990, Brain. Res. Bull. 25, 221-227).

Other neurotransmitters include the well known acetylcholine, norepinephrine which interacts with adrenergic receptors and which is regulated by tyrosine hydroxylase and monoamine oxidase, endorphins which are polypeptides that activate many central neurons and interact with opioid receptors, enkephalins, dynorphins, histamine, vasopressin, vasoactive intestinal peptide, carnosine, bradykinin, cholecystokinin, bombesin, somatostatin, corticotropin releasing factor, neurotensin, and adenosine.

As mentioned above, any imbalance in these neurotransmitters or any deregulation of associated receptors may lead to the development of CNS disorders ranging from psychiatric diseases to migraine, pain, memory loss and nerve cells degeneracy.

As of today, available treatments include selective serotonin reuptake inhibitors (SSRIs) such as fluoxetine, sertraline, paroxetine, and fluvoxamine. Other compounds include nefazodone which blocks the 5-HT2 receptor and inhibits reuptake of 5-HT and norepinephrine, trazodone which is a 5-HT2 receptor blocker and a 1-noradrenergic blocker, mirtazapine which blocks 2-adrenergic autoreceptors as well as 5-HT2, 5-HT3 and H1 receptors, tricyclic compounds such as imipramine and desipramine, tetracyclic compounds which increase the level of free norepinephrine and of 5-HT, and monoamine oxidase inhibitors (MAOI) which inhibit the oxidative deamination of norepinephrine, dopamine, and 5-HT. We can also cite lithium-antidepressants for treating bipolar disorder.

However, these compounds are only effective in about 65% of depressed patients, which implies a large population afflicted with the so-called "refractory depression". In some cases, the life of patients is in jeopardy at the extent that hospitalization and electroconvulsive therapy is required. This shows the seriousness of these diseases. Furthermore, the above mentioned compounds display numerous side effect such as tachycardia, sedation and weight gain.

Schizophrenia is also a serious mental disorder affecting about 1% of western countries population. Antipsychotic (neuroleptic) drugs available include chlorpromazine and haloperidol which show affinity for the dopamine 2 receptor. But, adverse side effects such as sedation, dystonia, tremors and akathisia have been commonly observed and a significant percentage of patients do not respond to the treatments.

Therefore, the problem is to find alternative solutions to provide a relief and a cure for the numerous patients afflicted with these diseases.

In connection with the present invention, we propose that mast cells are involved in or contribute to CNS disorders. Mast cells (MC) are tissue elements derived from a particular subset of hematopoietic stem cells that express CD34, c-kit and CD13 antigens (Kirshenbaum et al, Blood. 94: 2333-2342, 1999 and Ishizaka et al, Curr Opin Immunol. 5: 937-43, 1993). Immature MC progenitors circulate in the bloodstream and differentiate in tissues. These differentiation and proliferation processes are under the influence of cytokines, one of utmost importance being Stem Cell Factor (SCF), also termed Kit ligand (KL), Steel factor (SL) or Mast Cell Growth Factor (MCGF). SCF receptor is encoded by the protooncogene c-kit, that belongs to type III receptor tyrosine kinase subfamily (Boissan and Arock, J Leukoc Biol. 67: 135-48, 2000). This receptor is also expressed on others hematopoietic or non hematopoietic cells. Ligation of c-kit receptor by SCF induces its dimerization followed by its transphosphorylation, leading to the recruitement and activation of various intracytoplasmic substrates. These activated substrates induce multiple intracellular signaling pathways responsible for cell proliferation and activation (Boissan and Arock, 2000). Mast cells are characterized by their heterogeneity, not only regarding tissue location and structure but also at the functional and histochemical levels (Aldenborg and Enerback., Histochem. J. 26: 587-96, 1994 ; Bradding et al. J Immunol. 155: 297-307, 1995 ; Irani et al, J Immunol. 147: 247-53, 1991 ; Miller et al, Curr Opin Immunol. 1: 637-42, 1989 and Welle et al, J Leukoc Biol. 61: 233-45, 1997).

Here, it is postulated that the activation of mast cells by different stimuli such as stress, trauma, infection as well as neurotransmitters, participate in the exacerbation of the chemical imbalance causing CNS disorders.

More specifically, mast cell degranulation is stimulated by common neurotransmitters such as neurotensin, somatostatin, substance P and acetylcholine, by growth or survival factors, notably NGF, TGFβ1 (see figure 1). Mast cells involved in the response to such stimulus can be brain mast cells but also other mast cells releasing the content of their granules in the blood stream that ultimately reach sensory, motor or brain neurons. Brain mast cells staining is CTMC staining-like but they show the secretory pattern of MMC, implying that they constitute a particular subset of mast cells presenting specificities.

Following mast cells activation, released granules liberate various factors capable of modulating and altering neurotransmission and neurons survival. Among such factors, serotonin is important since an increase of the level of free serotonin has been observed in depressed patients. Alternatively, the sudden burst of serotonin may be followed by a period of serotonin shortage, leading to pain and migraine. As a consequence, we believe that mast cells exacerbate in autocrine or paracrine manner the deregulation of neurotransmission. For example, anxiety or stress-induced release of neurotransmitters such as serotonin activates mast cells, which in turn release the content of their granules, further contributing to the chemical imbalance in the brain leading to CNS disorders. Other mediators released by mast cells can be categorized into vasoactive, nociceptive, proinflammatory and other neurotransmitters. Taken together, these factors are able to induce great disturbance in the activity of neurons, whether they are sensory, motor, or CNS neurons.

We also observed that patients afflicted with mastocytosis are more incline to develop CNS disorders than the normal population. This can be explained by the presence of activating mutations in the c-kit receptor, which induce degranulation of mast cells and a burst of factors contributing to chemical imbalance and neurotransmission alteration.

In some cases, activated mast cells can also participate in the destruction of neuronal tissues by releasing a cocktail of different proteases and mediators categorized into three groups: preformed granule-associated mediators (histamine, proteoglycans, and neutral proteases), lipid-derived mediators (prostaglandins, thromboxanes and leucotrienes), and various cytokines (IL-1, IL-2, IL-3, IL-4, IL-5, IL-6, IL-8, TNF-α, GM-CSF, MIP-1α, MIP-1b, MIP-2 and IFN-γ). Then, liberation by activated mast cells of mediators (TNF-α, histamine, leucotrienes, prostaglandines etc...) as well as proteases is proposed here i) to induce inflammation and vasodilatation and ii) to participate in the neuronal tissue destruction process.

As a consequence, the present invention proposes to deplete mast cells using compounds that are substantially specific to mast cells. In this regard, tyrosine kinase inhibitors and more particularly c-kit specific kinase inhibitors are proposed to inhibit mast cell proliferation, survival and activation.

A new route for treating CNS disorders is provided, which consists of destroying mast cells involved in and contributing to the pathogenesis of these disorders. It has been found that tyrosine kinase inhibitors and more particularly c-kit inhibitors are especially suited to reach this goal.

### Description

The present invention relates to a method for treating CNS disorders comprising administering a compound capable of depleting mast cells to a human in need of such treatment.

Said method for treating CNS disorders can comprise administering a tyrosine kinase inhibitor to a human in need of such treatment.

Tyrosine kinase inhibitors are selected for example from bis monocyclic, bicyclic or heterocyclic aryl compounds (WO 92/20642), vinylene-azaindole derivatives (WO 94/14808) and 1-cycloproppyl-4-pyridyl-quinolones (US 5,330,992), Styryl compounds (US 5,217,999), styryl-substituted pyridyl compounds (US 5,302,606), seleoindoles and selenides (WO 94/03427), tricyclic polyhydroxylic compounds (WO 92/21660) and benzylphosphonic acid compounds (WO 91/15495), pyrimidine derivatives (US 5,521,184 and WO 99/03854), indolinone derivatives and pyrrol-substituted indolinones (US 5,792,783, EP 934 931, US 5,834,504, US 5,883,116, US 5,883,113, US 5, 886,020, WO 96/40116 and WO 00/38519), as well as bis monocyclic, bicyclic aryl and heteroaryl compounds (EP 584 222, US 5,656,643 and WO 92/20642), quinazoline derivatives (EP 602 851, EP 520 722, US 3,772,295 and US 4,343,940) and aryl and heteroaryl quinazoline (US 5,721,237, US 5,714,493, US 5,710,158 and WO 95/15758).

Preferably, said tyrosine kinase inhibitors are unable to promote death of IL-3 dependent cells cultured in presence of IL-3.

In another embodiment, the invention is directed to a method for treating CNS disorders comprising administering a c-kit inhibitor to a human in need of such treatment.

Preferably, said c-kit inhibitor is a non-toxic, selective and potent c-kit inhibitor. Such inhibitors can be selected from the group consisting of indolinones, pyrimidine derivatives, pyrrolopyrimidine derivatives, quinazoline derivatives, quinoxaline derivatives, pyrazoles derivatives, bis monocyclic, bicyclic or heterocyclic aryl compounds, vinylene-azaindole derivatives and pyridyl-quinolones derivatives, styryl compounds, styryl-substituted pyridyl compounds, seleoindoles, selenides, tricyclic polyhydroxylic compounds and benzylphosphonic acid compounds.

Among preferred compounds, it is of interest to focus on pyrimidine derivatives such as N-phenyl-2-pyrimidine-amine derivatives (US 5,521,184 and WO 99/03854), indolinone derivatives and pyrrol-substituted indolinones (US 5,792,783, EP 934 931, US 5,834,504), US 5,883,116, US 5,883,113, US 5, 886,020, WO 96/40116 and WO 00/38519), as well as bis monocyclic, bicyclic aryl and heteroaryl compounds (EP 584 222, US 5,656,643 and WO 92/20642), quinazoline derivatives (EP 602 851, EP 520 722, US 3,772,295 and US 4,343,940), 4-amino-substituted quinazolines (US 3,470,182), 4-thienyl-2-(1H)-quinazolones, 6,7-dialkoxyquinazolines (US 3,800,039), aryl and heteroaryl quinazoline (US 5,721,237, US 5,714,493, US 5,710,158 and WO 95/15758), 4-anilinoquinazoline compounds (US 4,464,375), and 4-thienyl-2-(1H)-quinazolones (US 3,551,427).

So, preferably, the invention relates to a method for treating CNS disorders comprising administering a non toxic, potent and selective c-kit inhibitor is a pyrimidine derivatives, more particularly N-phenyl-2-pyrimidine-amine derivatives of formula I : 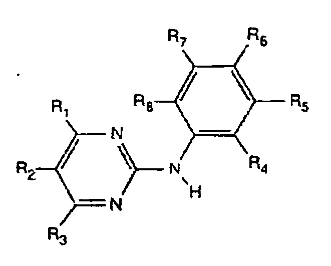 wherein
R1 is pyrazinyl, 1-methyl-1H-pyrrolyl, amino- or amino-lower alkyl-substituted phenyl wherein the amino group in each case is free or is in the form of lower alkylamino, dilower alkylamino, lower alkanoylamino or benzoylamino, 1H-indolyl or 1H-imidazolyl bonded at a five-membered ring carbon atom, or unsubstituted or lower alkyl-substituted pyridyl bonded at a ring carbon atom and unsubstituted or substituted at the nitrogen atom by oxygen,
R2 and R3 are each independently of the other hydrogen or lower alkyl,
one or two of the radicals R4, R5, R6, R7 and R8 are each nitro, fluoro-substituted lower alkoxy or a radical of formula II

-N(R9)-C(=X)-(Y)n-R10 (II),

wherein
R9 is hydrogen or lower alkyl,
X is oxo, thio, imino, N-lower alkyl-imino, hydroximino or O-lower alkyl-hydroximino,
Y is oxygen or the group NH,
n is 0 or 1 and
R10 is an aliphatic hydrocarbon radical having 5-22 carbon atoms, a phenyl or naphthyl radical each of which is unsubstituted or substituted by cyano, trifluoromethyl, hydroxy, lower alkoxy, lower alkanoyloxy, halogen, amino, lower alkylamino, di-lower alkylamino, lower alkanoylamino, benzoylamino, carboxy, lower alkoxycarbonyl or by unsubstituted or substituted lower alkyl, or phenyl-lower alkyl wherein the phenyl radical is unsubstituted or substituted as indicated above, a cycloalkyl or cycloalkenyl radical having up to 30 carbon atoms, cycloalkyl-lower alkyl or cycloalkenyl-lower alkyl each having up to 30 carbon atoms in the cycloalkyl or cycloalkenyl moiety, a monocyclic radical having 5 or 6 ring members and 1-3 ring atoms selected from nitrogen, oxygen and sulfur, to which radical one or two benzene radicals may be fused, or lower alkyl substituted by such a monocyclic radical, and the remaining radicals R4, R5, R6, R7 and R8 are each independently of the others hydrogen, lower alkyl that is unsubstituted or substituted by amino, lower alkylamino, di-lower alkylamino, piperazinyl, piperidinyl, pyrrolidinyl or by morpholinyl, or lower alkanoyl, trifluoromethyl, hydroxy, lower alkoxy, lower alkanoyloxy, halogen, amino, lower alkylamino, di-lower alkylamino, lower alkanoylamino, benzoylamino, carboxy or lower alkoxycarbonyl, the term "lower" in each case denoting a radical having up to and including 7 carbon atoms, or a salt of such a compound having at least one salt-forming group.

Preferably, the N-phenyl-2-pyrimidine-amine derivative is selected from the compounds corresponding to formula II : 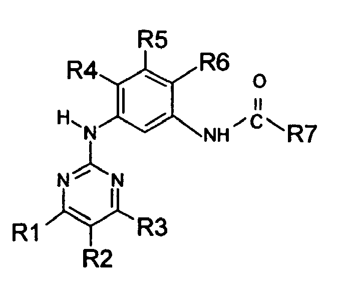 Wherein
R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one basic site, such as an amino function.

Preferably, R7 is the following group :

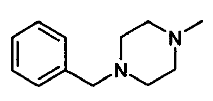

Among these compounds, the preferred are defined as follows :
R1 is a heterocyclic group, especially a pyridyl group,
R2 and R3 are H,
R4 is a C1-C3 alkyl, especially a methyl group,
R5 and R6 are H,
and R7 is a phenyl group bearing at least one substituent, which in turn possesses at least one
   basic site, such as an amino function, for example the group :

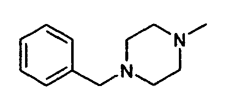

Therefore, in a preferred embodiment, the invention relates to a method for treating CNS disorders comprising the administration of an effective amount of the compound known in the art as CGP57148B :
4-(4-méhylpiperazine-1-ylméthyl)-N-[4-methyl-3-(4-pyridine-3-yl)pyrimidine-2 ylamino)phényl]-benzamide corresponding to the following formula:

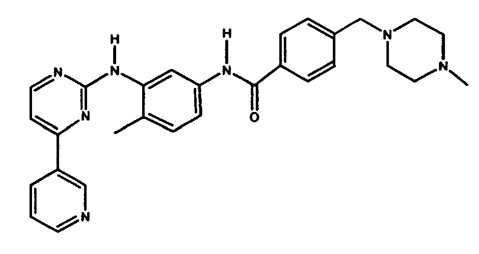

The preparation of this compound is described in example 21 of EP 564 409 and the β-form, which is particularly useful is described in WO 99/03854.

Alternatively, the c-kit inhibitor can be selected from :
- indolinone derivatives, more particularly pyrrol-substituted indolinones,
- monocyclic, bicyclic aryl and heteroaryl compounds, quinazoline derivatives,
- and quinaxolines, such as 2-phényl-quinaxoline derivatives, for example 2-phenyl-6,7-dimethoxy quinaxoline.

In a preferred aspect, the invention contemplated the method mentioned above, wherein said c-kit inhibitor is unable to promote death of IL-3 dependent cells cultured in presence of IL-3.

The CNS disorders as referred herein include but are not limited to psychiatric disorders, migraine, pain, memory loss and nerve cells degeneracy.

More particularly, the method according to the invention is useful for the treatment of the following disorders :
- Depression including dysthymic disorder, cyclothymic disorder, bipolar depression, severe or "melancholic" depression, atypical depression, refractory depression, seasonal depression, anorexia, bulimia, premenstrual syndrome, post-menopause syndrome.
- Other syndromes such as mental slowing and loss of concentration, pessimistic worry, agitation, self-deprecation, decreased libido,
- Pain including, acute pain, postoperative pain, chronic pain, nociceptive pain, cancer pain, neuropathic pain, psychogenic pain syndromes,
- Anxiety disorders including anxiety associated with hyperventilation and cardiac arrhythmias, phobic disorders, obsessive-compulsive disorder, posttraumatic stress disorder, acute stress disorder, generalized anxiety disorder,
- Psychiatric emergencies such as panic attacks, including psychosis, delusional disorders, conversion disorders, phobias, mania, delirium, dissociative episodes including dissociative amnesia, dissociative fugue and dissociative identity disorder, depersonalization, catatonia, seizures
- Severe psychiatric emergencies including suicidal behaviour, self-neglect, violent or aggressive behaviour, trauma, borderline personality, and acute psychosis,
- Schizophrenia including paranoid schizophrenia, disorganized schizophrenia, catatonic schizophrenia, and undifferentiated schizophrenia,
- Neurodegenerative diseases including Alzheimer's disease , Parkinson's disease, Huntington's disease, the prion diseases, Motor Neurone Disease (MND), and Amyotrophic Lateral Sclerosis (ALS).

Therefore, in a preferred embodiment, the method of the invention is applicable to the treatment of depression.

In another preferred embodiment, the method of the invention is applicable to the treatment of pain.

In another preferred embodiment, the method of the invention is applicable to the treatment of anxiety disorders.

In another preferred embodiment, the method of the invention is applicable to the treatment of psychiatric disorders.

In another preferred embodiment, the method of the invention is applicable to the treatment of schizophrenia.

In another preferred embodiment, the method of the invention is applicable to the treatment of neurodegenerative diseases.

The method as depicted above is also useful for treating memory loss.

In still another preferred embodiment, the method of the invention is applicable to the treatment of migraine.

In a further embodiment, c-kit inhibitors as mentioned above are inhibitors of activated c-kit. In frame with the invention, the expression "activated c-kit" means a constitutively activated-mutant c-kit including at least one mutation selected from point mutations, deletions, insertions, but also modifications and alterations of the natural c-kit sequence (SEQ ID N° 1). Such mutations, deletions, insertions, modifications and alterations can occur in the transphosphorylase domain, in the juxtamembrane domain as well as in any domain directly or indirectly responsible for c-kit activity. The expression "activated c-kit" also means herein SCF-activated c-kit. Preferred and optimal SCF concentrations for activating c-kit are comprised between 5.10⁻⁷ M and 5.10⁻⁶ M, preferably around 2.10⁻⁶ M. In a preferred embodiment, the activated-mutant c-kit in step a) has at least one mutation proximal to Y823, more particularly between amino acids 800 to 850 of SEQ ID No I involved in c-kit autophosphorylation, notably the D816V, D816Y, D816F and D820G mutants. In another preferred embodiment, the activated-mutant c-kit in step a) has a deletion in the juxtamembrane domain of c-kit. Such a deletion is for example between codon 573 and 579 called c-kit d(573-579). The point mutation V559G proximal to the juxtamembrane domain c-kit is also of interest.

In this regard, the invention contemplates a method for treating CNS disorders as defined above comprising administering to a human in need of such treatment a compound that is a selective, potent and non toxic inhibitor of activated c-kit obtainable by a screening method which comprises :
a) bringing into contact (i) activated c-kit and (ii) at least one compound to be tested; under conditions allowing the components (i) and (ii) to form a complex,
b) selecting compounds that inhibit activated c-kit,
c) testing and selecting a subset of compounds identified in step b), which are unable to promote death of IL-3 dependent cells cultured in presence of IL-3.

This screening method can further comprise the step consisting of testing and selecting a subset of compounds identified in step b) that are inhibitors of mutant activated c-kit (for example in the transphosphorylase domain), which are also capable of inhibiting SCF-activated c-kit wild.
Alternatively, in step a) activated c-kit is SCF-activated c-kit wild.

A best mode for practicing this method consists of testing putative inhibitors at a concentration above 10 µM in step a). Relevant concentrations are for example 10, 15, 20, 25, 30, 35 or 40 µM.

In step c), IL-3 is preferably present in the culture media of IL-3 dependent cells at a concentration comprised between 0.5 and 10 ng/ml, preferably between 1 to 5 ng/ml.

Examples of IL-3 dependent cells include but are not limited to :
- cell lines naturally expressing and depending on c-kit for growth and survival. Among such cells, human mast cell lines can be established using the following procedures :
   normal human mast cells can be infected by retroviral vectors containing sequences coding for a mutant c-kit comprising the c-kit signal peptide and a TAG sequence allowing to differentiate mutant c-kits from c-kit wild expressed in hematopoetic cells by means of antibodies.

This technique is advantageous because it does not induce cellular mortality and the genetic transfer is stable and gives satisfactory yields (around 20 %). Pure normal human mast cells can be routinely obtained by culturing precursor cells originating from blood obtained from human umbilical vein. In this regard, heparinated blood from umbilical vein is centrifuged on a Ficoll gradient so as to isolate mononucleated cells from other blood components. CD34+ precursor cells are then purified from the isolated cells mentioned above using the immunomagnetic selection system MACS (Miltenyi biotech). CD34+ cells are then cultured at 37°C in 5 % CO₂ atmosphere at a concentration of 10⁵ cells per ml in the medium MCCM (α-MEM supplemented with L-glutamine, penicillin, streptomycin, 5 10⁻⁵ M β-mercaptoethanol, 20 % veal foetal serum, I % bovine albumin serum and 100 ng/ml recombinant human SCF. The medium is changed every 5 to 7 days. The percentage of mast cells present in the culture is assessed each week, using May-Grünwal Giemsa or Toluidine blue coloration. Anti-tryptase antibodies can also be used to detect mast cells in culture. After 10 weeks of culture, a pure cellular population of mast cells (> 98 %) is obtained.

It is possible using standard procedures to prepare vectors expressing c-kit for transfecting the cell lines established as mentioned above. The cDNA of human c-kit has been described in Yarden et al., (1987) EMBO J.6 (11), 3341-3351. The coding part of c-kit (3000 bp) can be amplified by PCR and cloned, using the following oligonucleotides :
- 5'AAGAAGAGATGGTACCTCGAGGGGTGACCC3' (SEQ ID No2) sens
- 5'CTGCTTCGCGGCCGCGTTAACTCTTCTCAACCA3' (SEQ ID No3) antisens

The PCR products, digested with Not1 and Xho1, has been inserted using T4 ligase in the pFlag-CMV vector (SIGMA), which vector is digested with Not1 and Xho1 and dephosphorylated using CIP (Biolabs). The pFlag-CMV-c-kit is used to transform bacterial clone XL1-blue. The transformation of clones is verified using the following primers :
- 5'AGCTCGTTTAGTGAACCGTC3' (SEQ ID No4) sens,
- 5'GTCAGACAAAATGATGCAAC3' (SEQ ID No5) antisens.

Directed mutagenesis is performed using relevant cassettes is performed with routine and common procedure known in the art..
The vector Migr-1 (ABC) can be used as a basis for constructing retroviral vectors used for transfecting mature mast cells. This vector is advantageous because it contains the sequence coding for GFP at the 3' and of an IRES. These features allow to select cells infected by the retrovirus using direct analysis with a fluorocytometer. As mentioned above, the N-terminal sequence of c-kit c-DNA can be modified so as to introduce a Flag sequence that will be useful to discriminating heterogeneous from endogenous c-kit.

Other IL-3 dependent cell lines that can be used include but are not limited to:
- BaF3 mouse cells expressing wild-type or mutated form of c-kit (in the juxtamembrane and in the catalytic sites) are described in Kitayama et al, (1996), Blood 88, 995-1004 and Tsujimura et al, (1999), Blood 93, 1319-1329.
- IC-2 mouse cells expressing either c-kit^{WT} or c-kit^{D814Y} are presented in Piao et al, (1996), Proc. Natl. Acad. Sci. USA 93, 14665-14669.

IL-3 independent cell lines are :
- HMC-1, a factor-independent cell line derived from a patient with mast cell leukemia, expresses a juxtamembrane mutant c-kit polypeptide that has constitutive kinase activity (Furitsu T et al, J Clin Invest. 1993;92:1736-1744 ; Butterfield et al, Establishment of an immature mast cell line from a patient with mast cell leukemia. Leuk Res. 1988;12:345-355 and Nagata et al, Proc Natl Acad Sci U S A. 1995;92:10560-10564).
- P815 cell line (mastocytoma naturally expressing c-kit mutation at the 814 position) has been described in Tsujimura et al, (1994), Blood 83, 2619-2626.

The extent to which component (ii) inhibits activated c-kit can be measured *in vitro* or *in vivo.* In case it is measured *in vivo,* cell lines expressing an activated-mutant c-kit, which has at least one mutation proximal to Y823, more particularly between amino acids 800 to 850 of SEQ ID No1 involved in c-kit autophosphorylation, notably the D816V, D816Y, D816F and D820G mutants, are preferred.
Example of cell lines expressing an activated-mutant c-kit are as mentioned above.

In another preferred embodiment, the method further comprises the step consisting of testing and selecting compounds capable of inhibiting c-kit wild at concentration below 1 µM. This can be measured *in vitro* or *in vivo.*

Therefore, compounds are identified and selected according to the method described above are potent, selective and non-toxic c-kit wild inhibitors.

Alternatively, the screening method as defined above can be practiced *in vitro.* In this regard, the inhibition of mutant-activated c-kit and/or c-kit wild can be measured using standard biochemical techniques such as immunoprecipitation and western blot. Preferably, the amount of c-kit phosphorylation is measured.

In a still further embodiment, the invention contemplates a method for treating CNS disorders as depicted above wherein the screening comprises :
a) performing a proliferation assay with cells expressing a mutant c-kit (for example in the transphosphorylase domain), which mutant is a permanent activated c-kit, with a plurality of test compounds to identify a subset of candidate compounds targeting activated c-kit, each having an IC50 < 10 µM, by measuring the extent of cell death,
b) performing a proliferation assay with cells expressing c-kit wild said subset of candidate compounds identified in step (a), said cells being IL-3 dependent cells cultured in presence of IL-3, to identify a subset of candidate compounds targeting specifically c-kit,
c) performing a proliferation assay with cells expressing c-kit, with the subset of compounds identified in step b) and selecting a subset of candidate compounds targeting c-kit wild, each having an IC50 < 10 µM, preferably an IC50 < 1 µM, by measuring the extent of cell death.

Here, the extent of cell death can be measured by 3H thymidine incorporation, the trypan blue exclusion method or flow cytometry with propidium iodide. These are common techniques routinely practiced in the art.

The method according to the invention includes preventing, delaying the onset and/or treating CNS disorders in humans. Regarding the prion diseases, the invention encompasses the treatment of mammals such as bovine and ovine species.

In the method defined above, any compound capable of depleting mast cells can be used. Such compounds can belong to, as explicated above, tyrosine kinase inhibitors, such as c-kit inhibitors, but are not limited to any particular family so long as said compound shows capabilities to deplete mast cells. Depletion of mast cells can be evaluated using for example one of the mast cell lines depicted above using routine procedure.
Best compounds are compounds exhibiting the greatest selectivity.
Control cell lines include other hematopoeitic cells that are not mast cells or related cells or cell lines. These control cell lines include SCF independent expanded human CD34+ normal cells. These control cells also include but are not limited to the human T lymphocyte Jurkat cell line (ATCC N° TIB-152 and mutant cell lines derived thereof), the human B lymphocyte Daudi or Raji cell line (ATCC N° CCL-213 and CCL-86 respectively), the human monocytic U 937 cell line (ATCC N° CRL-1593.2) and the human HL-60 cell line (ATCC N° CCL-240) and mutant cell lines derived thereof CRL-2258 and CRL-2392).

Such compounds can be selected with a method for identifying compounds capable of depleting mast cells, said compound being non-toxic for cell types other than mast cells, comprising the step consisting of :
a) culturing mast cells in vitro in a culture medium suitable for mast cells,
b) adding to said culture medium at least one compound to be tested and incubating said cells for a prolonged period of time,
c) selecting compounds that promote mast cells death,
d) identifying a subset of compounds selected in step c) that are unable to promote death of cells selected from the above mentioned control cell lines.

Therefore, the invention embraces the use of the compounds defined above to manufacture a medicament for treating CNS disorders such as psychiatric disorders, migraine, pain, memory loss and nerve cells degeneracy.

The invention is also directed to the use of the compounds defined above to manufacture a medicament for treating a disorders selected from the subgroups consisting of :
- Depression including dysthymic disorder, cyclothymic disorder, bipolar depression, severe or "melancholic" depression, atypical depression, seasonal depression, anorexia, bulimia, premenstrual syndrome, post-menopause syndrome.
- Other syndromes such as mental slowing and loss of concentration, pessimistic worry, agitation, self-deprecation, decreased libido,
- Pain including, acute pain, postoperative pain, chronic pain, nociceptive pain, cancer pain, neuropathic pain, psychogenic pain syndromes,
- Anxiety disorders including anxiety associated with hyperventilation and cardiac arrhythmias, phobic disorders, obsessive-compulsive disorder, posttraumatic stress disorder, acute stress disorder, generalized anxiety disorder,
- Psychiatric emergencies such as panic attacks, including psychosis, delusional disorders, conversion disorders, phobias, mania, delirium, dissociative episodes including dissociative amnesia, dissociative fugue and dissociative identity disorder, depersonalization, catatonia, seizures
- Severe psychiatric emergencies including suicidal behaviour, self-neglect, violent or aggressive behaviour, trauma, borderline personality, and acute psychosis,
- Schizophrenia including paranoid schizophrenia, disorganized schizophrenia, catatonic schizophrenia, and undifferentiated schizophrenia,
- Neurodegenerative diseases including Alzheimer's disease , Parkinson's disease, Huntington's disease, the prion diseases, Motor Neurone Disease (MND), and Amyotrophic Lateral Sclerosis (ALS).

The pharmaceutical compositions utilized in this invention may be administered by any number of routes including, but not limited to, oral, intravenous, intramuscular, intraarterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

More particularly, the invention relates to a pharmaceutical composition intended for oral administration.

Regarding the treatment of pain, topical administration may be most suitable in some cases. Here, the compositions according to the invention may be presented in the form of a gel, paste, ointment, cream, lotion, liquid suspension aqueous, aqueous-alcoholic or, oily solutions, or dispersions of the lotion or serum type, or anhydrous or lipophilic gels, or emulsions of liquid or semi-solid consistency of the milk type, obtained by dispersing a fatty phase in an aqueous phase or vice versa, or of suspensions or emulsions of soft, semi-solid consistency of the cream or gel type, or alternatively of microemulsions, of microcapsules, of microparticles or of vesicular dispersions to the ionic and/or nonionic type. These compositions are prepared according to standard methods.

The composition according to the invention comprises any ingredient commonly used in dermatology and cosmetic. It may comprise at least one ingredient selected from hydrophilic or lipophilic gelling agents, hydrophilic or lipophilic active agents, preservatives, emollients, viscosity enhancing polymers, humectants, surfactants, preservatives, antioxidants, solvents, and fillers, antioxidants, solvents, perfumes, fillers, screening agents, bactericides, odor absorbers and coloring matter.

As oils which can be used in the invention, mineral oils (liquid paraffin), vegetable oils (liquid fraction of shea butter, sunflower oil), animal oils, synthetic oils, silicone oils (cyclomethicone) and fluorinated oils may be mentioned. Fatty alcohols, fatty acids (stearic acid) and waxes (paraffin, carnauba, beeswax) may also be used as fatty substances.

As emulsifiers which can be used in the invention, glycerol stearate, polysorbate 60 and the PEG-6/PEG-32/glycol stearate mixture are contemplated.
As hydrophilic gelling agents, carboxyvinyl polymers (carbomer), acrylic copolymers such as acrylate/alkylacrylate copolymers, polyacrylamides, polysaccharides such as hydroxypropylcellulose, clays and natural gums may be mentioned, and as lipophilic gelling agents, modified clays such as bentones, metal salts of fatty acids such as aluminum stearates and hydrophobic silica, or alternatively ethylcellulose and polyethylene may be mentioned.

As hydrophilic active agents, proteins or protein hydrolysates, amino acids, polyols, urea, allantoin, sugars and sugar derivatives, vitamins, starch and plant extracts, in particular those of Aloe vera may be used.

As lipophilic active, agents, retinol (vitamin A) and its derivatives, tocopherol (vitamin E) and its derivatives, essential fatty acids, ceramides and essential oils may be used. These agents add extra moisturizing or skin softening features when utilized.

In addition, a surfactant can be included in the composition so as to provide deeper penetration of the compound capable of depleting mast cells, such as a tyrosine kinase inhibitor, preferably a c-kit inhibitor.

Among the contemplated ingredients, the invention embraces penetration enhancing agents selected for example from the group consisting of mineral oil, water, ethanol, triacetin, glycerin and propylene glycol; cohesion agents selected for example from the group consisting of polyisobutylene, polyvinyl acetate and polyvinyl alcohol, and thickening agents.

Chemical methods of enhancing topical absorption of drugs are well known in the art. For example, compounds with penetration enhancing properties include sodium lauryl sulfate (Dugard, P. H. and Sheuplein, R. J., "Effects of Ionic Surfactants on the Permeability of Human Epidermis: An Electrometric Study," J. Ivest. Dermatol., V.60, pp. 263-69, 1973), lauryl amine oxide (Johnson et. al., US 4,411,893), azone (Rajadhyaksha, US 4,405,616 and 3,989,816) and decylmethyl sulfoxide (Sekura, D. L. and Scala, J., "The Percutaneous Absorption of Alkylmethyl Sulfides," Pharmacology of the Skin, Advances In Biolocy of Skin, (Appleton-Century Craft) V. 12, pp. 257-69, 1972). It has been observed that increasing the polarity of the head group in amphoteric molecules increases their penetration-enhancing properties but at the expense of increasing their skin irritating properties (Cooper, E. R. and Berner, B., "Interaction of Surfactants with Epidermal Tissues: Physiochemical Aspects," Surfactant Science Series, V. 16, Reiger, M. M. ed. (Marcel Dekker, Inc.) pp. 195-210, 1987).

A second class of chemical enhancers are generally referred to as co-solvents. These materials are absorbed topically relatively easily, and, by a variety of mechanisms, achieve permeation enhancement for some drugs. Ethanol (Gale et. al., U.S. Pat. No. 4,615,699 and Campbell et. al., U.S. Pat. Nos. 4,460,372 and 4,379,454), dimethyl sulfoxide (US 3,740,420 and 3,743,727, and US 4,575,515), and glycerine derivatives (US 4,322,433) are a few examples of compounds which have shown an ability to enhance the absorption of various compounds.

Pharmaceutical compositions suitable for use in the invention include compositions wherein compounds for depleting mast cells, such as tyrosine kinase inhibitors and c-kit inhibitors, are contained in an effective amount to achieve the intended purpose. The determination of an effective dose is well within the capability of those skilled in the art. A therapeutically effective dose refers to that amount of active ingredient, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions which exhibit large therapeutic indices are preferred. As mentioned above, a tyrosine kinase inhibitor and more particularly a c-kit inhibitor according to the invention is unable to promote death of IL-3 dependent cells cultured in presence of IL-3.

The invention also contemplates a product comprising at least one compound capable of depleting mast cells, such as a tyrosine kinase inhibitors, more particularly a non-toxic, selective and potent c-kit inhibitor and at least one antidepressant, antipsychotic, or anxiolytic for simultaneous, separate or sequential use for the treatment of CNS disorders as defined above.

## Claims

1. The use of a c-kit inhibitor for preparing a medicament for treating CNS disorders.

2. The use according to claim 1, wherein said inhibitor is selected from the group consisting of N-phenyl-2-pyrimidine-amine derivatives of formula I : 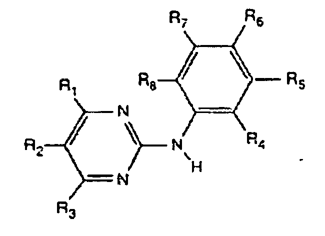 wherein
R1 is pyrazinyl, 1-methyl-1H-pyrrolyl, amino- or amino-lower alkyl-substituted phenyl wherein the amino group in each case is free or is in the form of lower alkylamino, dilower alkylamino, lower alkanoylamino or benzoylamino, 1H-indolyl or 1H-imidazolyl bonded at a five-membered ring carbon atom, or unsubstituted or lower alkyl-substituted pyridyl bonded at a ring carbon atom and unsubstituted or substituted at the nitrogen atom by oxygen,
R2 and R3 are each independently of the other hydrogen or lower alkyl,
one or two of the radicals R4, R5, R6, R7 and R8 are each nitro, fluoro-substituted lower alkoxy or a radical of formula II
-N(R9)-C(=x)-Mn-R10 (II),
wherein
R9 is hydrogen or lower alkyl,
X is oxo, thio, imino, N-lower alkyl-imino, hydroximino or O-lower alkyl-hydroximino,
Y is oxygen or the group NH,
n is 0 or 1 and
R10 is an aliphatic hydrocarbon radical having 5-22 carbon atoms, a phenyl or naphthyl radical each of which is unsubstituted or substituted by cyano, trifluoromethyl, hydroxy, lower alkoxy, lower alkanoyloxy, halogen, amino, lower alkylamino, di-lower alkylamino, lower alkanoylamino, benzoylamino, carboxy, lower alkoxycarbonyl or by unsubstituted or substituted lower alkyl, or phenyl-lower alkyl wherein the phenyl radical is unsubstituted or substituted as indicated above, a cycloalkyl or cycloalkenyl radical having up to 30 carbon atoms, cycloalkyl-lower alkyl or cycloalkenyl-lower alkyl each having up to 30 carbon atoms in the cycloalkyl or cycloalkenyl moiety, a monocyclic radical having 5 or 6 ring members and 1-3 ring atoms selected from nitrogen, oxygen and sulfur, to which radical one or two benzene radicals may be fused, or lower alkyl substituted by such a monocyclic radical,
and the remaining radicals R4, R5, R6, R7 and R8 are each independently of the others hydrogen, lower alkyl that is unsubstituted or substituted by amino, lower alkylamino, di-lower alkylamino, piperazinyl, piperidinyl, pyrrolidinyl or by morpholinyl, or lower alkanoyl, trifluoromethyl, hydroxy, lower alkoxy, lower alkanoyloxy, halogen, amino, lower alkylamino, di-lower alkylamino, lower alkanoylamino, benzoylamino, carboxy or lower alkoxycarbonyl, the term "lower" in each case denoting a radical having up to and including 7 carbon atoms, or a salt of such a compound having at least one salt-forming group.

3. The use according to claim 1, wherein said inhibitor is selected from the group consisting of N-phenyl-2-pyrimidine-amine derivatives having the formula II : 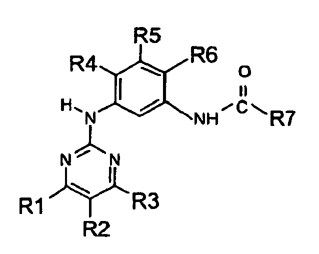 Wherein
R1 , R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one basic site, such as an amino function.

4. The use according to claim 3, wherein R7 is

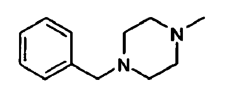

5. The use according to claim 3, wherein said inhibitor is the 4-(4-mehylpiperazin-1-ylmethyl)-N-[4-methyl-3-(4-pyridin-3-yl)pyrimidin-2 ylamino)phenyl]-benzamide.

6. The use according to claim 1, wherein the c-kit inhibitor is obtainable by a screening method comprising:
a) performing a proliferation assay with cells expressing a mutant c-kit (for example in the transphosphorylase domain), which mutant is a permanent activated c-kit, with a plurality of test compounds to identify a subset of candidate compounds targeting activated c-kit, each having an IC50 < 10 µM, by measuring the extent of cell death,
b) performing a proliferation assay with cells expressing c-kit wild said subset of candidate compounds identified in step (a), said cells being IL-3 dependent cells cultured in presence of IL-3, to identify a subset of candidate compounds targeting specifically c-kit,
c) performing a proliferation assay with cells expressing c-kit, with the subset of compounds identified in step b) and selecting a subset of candidate compounds targeting c-kit wild, each having an IC50 < 10 µM, preferably an IC50 < 1 µM, by measuring the extent of cell death.

7. The use according to one of claims 1 to 6 for preventing, delaying the onset and/or treating CNS disorders in human.

8. The use according to claim 7 for treating psychiatric disorders, migraine, pain, memory loss and nerve cells degeneracy.

9. The use according to claim 7 for treating depression including dysthymic disorder, cyclothymic disorder, bipolar depression, severe or "melancholic" depression, atypical depression, refractory depression, seasonal depression, anorexia, bulimia, premenstrual syndrome and post-menopause syndrome.

10. The use according to claim 7 for treating mental slowing and loss of concentration, pessimistic worry, agitation, self-deprecation and decreased libido.

11. The use according to claim 7 for treating pain including, acute pain, postoperative pain, chronic pain, nociceptive pain, cancer pain, neuropathic pain, and psychogenic pain syndromes.

12. The use according to claim 7 for treating anxiety disorders including anxiety associated with hyperventilation and cardiac arrhythmias, phobic disorders, obsessive-compulsive disorder, posttraumatic stress disorder, acute stress disorder, and generalized anxiety disorder.

13. The use according to claim 7 for treating psychiatric disorders such as panic attacks, including psychosis, delusional disorders, conversion disorders, phobias, mania, delirium, dissociative episodes including dissociative amnesia, dissociative fugue and dissociative identity disorder, depersonalization, catatonia, and seizures.

14. The use according to claim 7 for treating severe psychiatric disorders including suicidal behaviour, self-neglect, violent or aggressive behaviour, trauma, borderline personality, and acute psychosis.

15. The use according to claim 7 for treating schizophrenia including paranoid schizophrenia, disorganized schizophrenia, catatonic schizophrenia, and undifferentiated schizophrenia.

16. The use according to claim 7 for treating neurodegenerative diseases including Alzheimer's disease, Parkinson's disease, Iduntington's disease, the prion diseases, Motor Neurone Disease (MND), and Amyotrophic Lateral Sclerosis (ALS).

17. The use according to claim 7 for treating memory loss.

18. The use according to claim 7 for treating migraine.

19. The use according to claim 7 for the treatment of pain.

20. The use according to claim 19 wherein the medicament is suitable for topical administration.

## Patentansprüche

1. Verwendung eines c-kit-Inhibitors zur Herstellung eines Medikaments zur Behandlung von ZNS-Erkrankungen.

2. Verwendung gemäß Anspruch 1, wobei der Inhibitor ausgewählt ist aus N-Phenyl-2-pyrimidin-amin-Derivaten der Formel I: 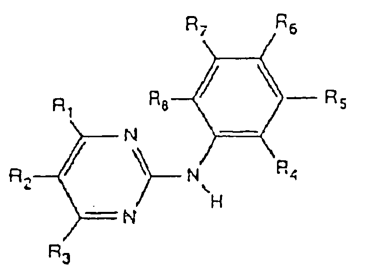 wobei
R1 Pyrazinyl, 1-Methyl-1H-pyrrolyl, Amino- oder Amino-niederes Alkyl-substituiertes Phenyl, wobei die Aminogruppe jeweils frei ist oder in Form eines niederen Alkylamino, di-niederen Alkylamino, niederen Alkanoylamino oder Benzoylamino vorliegt, 1H-Indolyl oder 1H-Imidazolyl, gebunden an ein Kohlenstoffatom eines fünfgliedrigen Rings, oder unsubstituiertes oder niederes Alkyl-substituiertes Pyridyl, gebunden an ein Kohlenstoffatom eines Rings und unsubstituiert oder am Stickstoffatom substituiert durch Sauerstoff, ist,
R2 und R3 jeweils unabhängig voneinander Wasserstoff oder ein niederes Alkyl sind,
einer oder zwei der Radikale R4, R5, R6, R7 und R8 jeweils Nitro, Fluorsubstituiertes niederes Alkoxy oder ein Radikal der Formel II sind
-N(R9)-C(=X)-(Y)n-R10 (II),
wobei
R9 Wasserstoff oder ein niederes Alkyl ist,
X Oxo, Thio, Imino, N-niederes Alkyl-imino, Hydroximino oder O-niederes Alkyl-hydroximino ist,
Y Sauerstoff oder die Gruppe NH ist,
n 0 oder 1 ist, und
R10 ein aliphatisches Kohlenwasserstoff-Radikal mit 5 bis 22 Kohlenstoffatomen, ein Phenyl- oder Naphthyl-Radikal, jeweils unsubstituiert oder substituiert durch Cyano, Trifluormethyl, Hydroxy, niederes Alkoxy, niederes Alkanoyloxy, Halogen, Amino, niederes Alkylamino, di-niederes Alkyl-amino, niederes Alkanoylamino, Benzoylamino, Carboxy, niederes Alkoxycarbonyl oder durch unsubstituiertes oder substituiertes niederes Alkyl oder Phenyl-niederes Alkyl, wobei das Phenyl-Radikal unsubstituiert oder wie oben aufgeführt substituiert ist, ein Cycloalkyl- oder Cycloalkenyl-Radikal mit bis zu 30 Kohlenstoffatomen, Cycloalkylniederes Alkyl oder Cycloalkenyl-niederes Alkyl mit jeweils bis zu 30 Kohlenstoffatomen in dem Cycloalkyl- oder Cycloalkenyl-Anteil, ein monozyklisches Radikal mit 5 oder 6 Ringbestandteilen und 1 bis 3 Ringatomen, ausgewählt aus Stickstoff, Sauerstoff und Schwefel, wobei an das Radikal ein oder zwei Benzol-Radikale gebunden sein können, oder niederes Alkyl, substituiert durch ein derartiges monozyklisches Radikal, ist,
und die übrigen Radikale R4, R5, R6, R7 und R8 jeweils unabhängig voneinander Wasserstoff, niederes Alkyl, welches unsubstituiert oder durch Amino, niederes Alkylamino, di-niederes Alkylamino, Piperazinyl, Piperidinyl, Pyrrolidinyl oder durch Morpholinyl substituiert ist, oder niederes Alkanoyl, Trifluormethyl, Hydroxy, niederes Alkoxy, niederes Alkanoyloxy, Halogen, Amino, niederes Alkylamino, di-niederes Alkylamino, niederes Alkanoylamino, Benzoylamino, Carboxy oder niederes Alkylcarbonyl sind, wobei der Ausdruck "nieder" in jedem Fall ein Radikal mit bis zu einschließlich 7 Kohlenstoffatomen bezeichnet, oder ein Salz einer derartigen Verbindung, welches wenigstens eine salzbildende Gruppe aufweist.

3. Verwendung gemäß Anspruch 1, wobei der Inhibitor ausgewählt ist aus N-Phenyl-2-pyrimidin-amin-Derivaten mit der Formel II: 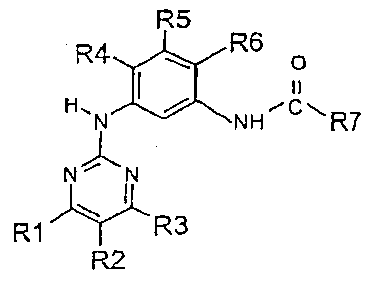 wobei
R1, R2 und R3 unabhängig voneinander ausgewählt sind aus H, F, Cl, Br, I, einer C1-C5-Alkylgruppe oder einer zyklischen oder heterozyklischen Gruppe, insbesondere einer Pyridylgruppe,
R4, R5 und R6 unabhängig voneinander ausgewählt sind aus H, F, Cl, Br, I, einer C1-C5-Alkylgruppe, insbesondere einer Methylgruppe,
und R7 eine Phenylgruppe ist, die zumindest einen Substituenten trägt, welcher wiederum zumindest eine basische Seite, wie beispielsweise eine Aminofunktion, aufweist.

4. Verwendung gemäß Anspruch 3, wobei R7 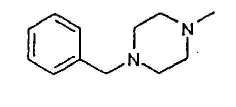 ist.

5. Verwendung gemäß Anspruch 3, wobei der Inhibitor 4-(4-Methylpiperazin-1-ylmethyl)-N-[4-methyl-3-(4-pyridin-3-yl)pyrimidin-2-ylamino)phenyl]-benzamid ist.

6. Verwendung gemäß Anspruch 1, wobei der c-kit-Inhibitor erhältlich ist durch ein Screening-Verfahren, welches folgende Stufen umfasst:
a) Durchführen eines Proliferations-Assays mit Zellen, die ein mutantes c-kit exprimieren (beispielsweise in der Transphosphorylase-Domäne), wobei die Mutante ein permanent aktiviertes c-kit ist, mit einer Vielzahl von Testverbindungen zur Identifizierung einer Untergruppe von Kandidat-Verbindungen, die auf aktiviertes c-kit abzielen, jeweils mit einem IC50 < 10 µM, durch Messen des Zelltod-Ausmaßes,
b) Durchführen eines Proliferations-Assays mit Zellen, die c-kit-wild(typ) exprimieren, mit der Untergruppe der in Stufe (a) identifizierten Kandidat-Verbindungen, wobei die Zellen IL-3-abhängige Zellen sind, die in Gegenwart von IL-3 kultiviert werden, zur Identifizierung einer Untergruppe von Kandidat-Verbindungen, die spezifisch auf c-kit abzielen,
c) Durchführen eines Proliferations-Assays mit Zellen, die c-kit exprimieren, mit der Untergruppe von in Stufe (b) identifizierten Verbindungen und Selektieren einer Untergruppe von Kandidat-Verbindungen, die auf c-kit-wild(typ) abzielen, jeweils mit einem IC50 < 10 µM, bevorzugt einem IC50 < 1 µM, durch Messen des Zelltod-Ausmaßes.

7. Verwendung gemäß einem der Ansprüche 1 bis 6 zur Vorbeugung, Verzögerung des Einsetzens und/oder Behandlung von ZNS-Erkrankungen beim Menschen.

8. Verwendung gemäß Anspruch 7 zur Behandlung von psychiatrischen Erkrankungen, Migräne, Schmerz, Gedächtnisverlust und Nervenzellen-Degeneration.

9. Verwendung gemäß Anspruch 7 zur Behandlung von Depression, einschließlich dysthymischer Erkrankung, cyclothymischer Erkrankung, bipolarer Depression, schwerer oder "melancholischer" Depression, atypischer Depression, refraktorischer Depression, saisonaler Depression, Anorexie, Bulimie, prämenstruellem Syndrom und Post-Monopausen-Syndrom.

10. Verwendung gemäß Anspruch 7 zur Behandlung von mentaler Verlangsamung, Konzentrationsverlust, pessimistischer Sorge, Unruhe, Selbstablehnung und verminderter Libido.

11. Verwendung gemäß Anspruch 7 zur Behandlung von Schmerz, einschließlich akutem Schmerz, postoperativem Schmerz, chronischem Schmerz, nozizeptivem Schmerz, Krebsschmerz, neuropatischem Schmerz und psychogenen Schmerzsyndromen.

12. Verwendung gemäß Anspruch 7 zur Behandlung von Angstzuständen, einschließlich Angst in Verbindung mit Hyperventilation und herzbezüglichen Arrhythmien, Phobie-Erkrankungen, obsessivkompulsiven Erkrankungen, posttraumatische Stresserkrankungen, akuten Stresserkrankungen und allgemeinen Angstzuständen.

13. Verwendung gemäß Anspruch 7 zur Behandlung von psychiatrischen Erkrankungen, wie beispielsweise Panikattacken, einschließlich Psychose, Delusions-Erkrankungen, Konversions-Erkrankungen, Phobien, Manien, Delirium, dissoziativen Episoden, einschließlich dissoziativer Amnesie, dissoziativer Poromanie und dissoziativer Identitätsstörung, Persönlichkeitsentfremdung, Katatonie und Anfällen.

14. Verwendung gemäß Anspruch 7 zur Behandlung schwerer psychiatrischer Erkrankungen, einschließlich Suizidverhalten, Selbstvernachlässigung, gewalttätigem oder aggressivem Verhalten, Trauma, Grenzlinien-Persönlichkeit und akuter Psychose.

15. Verwendung gemäß Anspruch 7 zur Behandlung von Schizophrenie, einschließlich paranoider Schizophrenie, disorganisierter Schizophrenie, katatonischer Schizophrenie und undifferenzierter Schizophrenie.

16. Verwendung gemäß Anspruch 7 zur Behandlung neurodegenerativer Erkrankungen, einschließlich Alzheimer-Krankheit, Parkinson-Krankheit, Huntington-Krankheit, Prion-Erkrankungen, Motor-Neurone-Disease (MND) und Amyotropher Lateralsklerose (ALS).

17. Verwendung gemäß Anspruch 7 zur Behandlung von Gedächtnisverlust.

18. Verwendung gemäß Anspruch 7 zur Behandlung von Migräne.

19. Verwendung gemäß Anspruch 7 zur Behandlung von Schmerz.

20. Verwendung gemäß Anspruch 19, wobei das Medikament zur topischen Verabreichung geeignet ist.

## Revendications

1. Utilisation d'un inhibiteur de c-kit pour préparer un médicament destiné à traiter les troubles du SNC.

2. Utilisation selon la revendication 1, dans laquelle ledit inhibiteur est choisi à partir du groupe comportant des dérivés N-phényl-2-pyrimidine-amine de formule I : 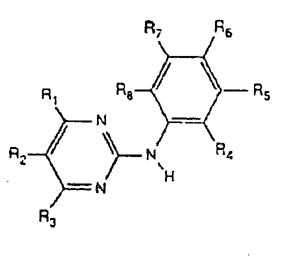 où
R1 est le pyrazinyle, le 1-méthyl-1H-pyrrolyle, un phényle substitué par un amino ou par un amino alkyle inférieur, dans lequel le groupe amino, dans chaque cas, est libre ou est sous forme d'un alkylamino inférieur, d'un dialkylamino inférieur, d'un alkanoylamino inférieur ou d'un benzoylamino, le 1H-indolyl ou le 1H-imidazolyle lié à un atome de carbone d'un cycle à 5 membres, ou un pyridyl non substitué ou substitué par un alkyle inférieur lié au niveau d'un atome de carbone de cycle et non substitué ou substitué au niveau de l'atome d'azote par de l'oxygène,
R2 et R3 sont chacun indépendamment de l'autre de l'hydrogène ou un alkyle inférieur,
un ou deux des radicaux R4, R5, R6, R7 et R8 sont chacun un alcoxy inférieur nitro ou fluoro-substitué ou un radical de formule II
-N(R9)-C(=X)-(Y)n-R10 (II),
où
R9 est de l'hydrogène ou un alkyle inférieur,
X est oxo, thio, imino, N-imino-alkyle inférieur, hydroxyimino ou O-hydroxyimino-alkyle inférieur,
Y est de l'oxygène ou le groupe NH,
N vaut 0 ou 1 et
R10 est un radical hydrocarboné aliphatique ayant de 5 à 22 atomes de carbone, un radical phényle ou naphtyle chacun étant non substitué ou substitué par un cyano, un trifluorométhyle, un hydroxy, un alcoxy inférieur, un alcanoyloxy inférieur, un halogène, un amino, un alkylamino inférieur, un di-alkylamino inférieur, un alcanoylamino inférieur, un benzoylamino, un carboxy, un alcoxycarbonyle inférieur ou par un alkyle inférieur non substitué ou substitué, ou un phényle-alkyle inférieur où le radical phényle est non substitué ou substitué comme indiqué ci-dessus, un radical cycloalkyle ou cycloalcényle ayant jusqu'à 30 atomes de carbone, un cycloalkyle-alkyle inférieur ou cycloalcényle-alkyle inférieur ayant chacun jusqu'à 30 atomes de carbone dans la fraction cycloalkyle ou cycloalcényle, un radical monocyclique ayant 5 ou 6 membres par cycle et 1 à 3 atomes de cycle choisis à partir de l'azote, de l'oxygène et du soufre, auquel radical un ou deux radicaux benzène peuvent fusionner, ou alkyle inférieur substitué par un tel radical monocyclique,
et le reste des radicaux R4, R5, R6, R7 et R8 sont chacun indépendamment des autres de l'hydrogène, un alkyle inférieur qui est non substitué ou substitué par un amino, un alkylamino inférieur, un di-alkylamino inférieur, le pipérazinyle, le pipéridinyle, le pyrrolidinyle ou par le morpholinyle, ou alcanoyle inférieur, trifluorométhyle, hydroxy, alcoxy inférieur, alcanoyloxy inférieur, halogène, amino, alkylamino inférieur, di-alkylamino inférieur, alkanoylamino inférieur, benzoylamino, carboxy ou alcoxycarbonyle inférieur, le terme « inférieur » dans chaque cas désignant un radical ayant un nombre d'atomes de carbone allant jusqu'à 7 y compris, ou un sel d'un tel composé ayant au moins un groupe formant des sels.

3. Utilisation selon la revendication 1, dans laquelle ledit inhibiteur est choisi à partir du groupe comportant les dérivés de N-phényl-2-pyrimidine-amine de formule II : 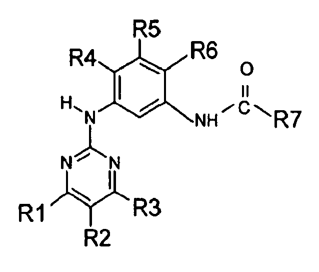 où
R1, R2 et R3 sont indépendamment choisis à partir de H, F, Cl, Br, I, un alkyle en C₁-C₅, ou un groupe cyclique ou hétérocyclique, en particulier un groupe pyridyle ;
R4, R5 et R6 sont indépendamment choisis à partir de H, F, Cl, Br, I, un alkyle en C₁-C₅, en particulier un groupe méthyle ;
et R7 est un groupe phényle portant au moins un substituant, qui à son tour possède au moins un site basique, comme une fonction amino.

4. Utilisation selon la revendication 3, dans laquelle R7 est

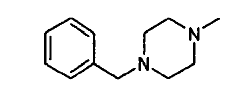

5. Utilisation selon la revendication 3, dans laquelle ledit inhibiteur est le 4-(4-méthylpipérazine-1-ylméthyl)-N-[4-méthyl-3-(4-pyridine-3-yl)pyrimidine-2-ylamino)phényl]-benzamide.

6. Utilisation selon la revendication 1, dans laquelle l'inhibiteur de c-kit peut être obtenu par un procédé de criblage comprenant les étapes consistant à :
a) effectuer une étude de prolifération avec des cellules exprimant un c-kit mutant (par exemple dans le domaine de la kinase), lequel mutant est un c-kit activé permanent, avec un certain nombre de composés tests de façon à identifier un sous-ensemble de composés candidats ciblant le c-kit activé, chacun ayant une CI50 < 10 µM, en mesurant l'étendue de la mort cellulaire,
b) effectuer une étude de prolifération avec des cellules exprimant le c-kit sauvage ledit sous-ensemble de composés candidats identifié à l'étape a), lesdites cellules étant des cellules dépendant des IL-3 mises en culture en présence d'IL-3, de façon à identifier un sous-ensemble de composés candidats ciblant spécifiquement le c-kit,
c) effectuer une étude de prolifération avec des cellules exprimant le c-kit, avec le sous-ensemble de composés identifiés à l'étape b) et sélectionner un sous-ensemble de composés candidats ciblant le c-kit sauvage, chacun ayant une IC50 < 10 µM, de préférence une IC50 < 1 µM, en mesurant l'étendue de la mort cellulaire.

7. Utilisation selon l'une quelconque des revendications 1 à 6 visant à prévenir, à retarder l'apparition et/ou à traiter les troubles du SNC chez l'homme.

8. Utilisation selon la revendication 7 visant à traiter les troubles psychiatriques, la migraine, la douleur, la perte de mémoire et la dégénérescence des neurones.

9. Utilisation selon la revendication 7 visant à traiter la dépression, y compris les troubles dysthymiques, les troubles cyclothymiques, la dépression bipolaire, la dépression grave ou « mélancolique », la dépression atypique, la dépression réfractaire, la dépression saisonnière, l'anorexie, la boulimie, le syndrome prémenstruel, et le syndrome postménopausique.

10. Utilisation selon la revendication 7 visant à traiter le ralentissement de l'activité intellectuelle et la perte de concentration, l'inquiétude pessimiste, l'agitation, l'auto-dévalorisation et la baisse de la libido.

11. Utilisation selon la revendication 7 visant à traiter la douleur y compris la douleur aiguë, la douleur postopératoire, la douleur chronique, la douleur nociceptive, la douleur consécutive à un cancer, la douleur neuropathique, et les syndromes de la douleur psychogène.

12. Utilisation selon la revendication 7 visant à traiter les troubles de l'angoisse, y compris l'angoisse associée à l'hyperventilation et aux arythmies cardiaques, aux troubles phobiques, aux troubles obsessionnels compulsifs, à l'état de stress post-traumatique, aux troubles du stress aigu et aux troubles de l'angoisse généralisée.

13. Utilisation selon la revendication 7 visant à traiter les troubles psychiatriques comme les crises de panique, y compris la psychose, les troubles délirants, les troubles de conversion, les phobies, les manies, le délire, les épisodes dissociatifs y compris l'amnésie dissociative, la fugue dissociative et le trouble d'identité dissociative, la dépersonnalisation, la catatonie et les crises.

14. Utilisation selon la revendication 7 visant à traiter les troubles psychiatriques graves y compris le comportement suicidaire, la négligence de soi, le comportement violent ou agressif, le traumatisme, la personnalité borderline, et la psychose aiguë.

15. Utilisation selon la revendication 7 visant à traiter la schizophrénie y compris la schizophrénie paranoïde, la schizophrénie désorganisée, la schizophrénie catatonique et la schizophrénie non différenciée.

16. Utilisation selon la revendication 7 visant à traiter les maladies neurodégénératives y compris la maladie d'Alzheimer, la maladie de Parkinson, la chorée de Huntington, la maladie du prion, l'affection du neurone moteur, la sclérose latérale amyotrophique.

17. Utilisation selon la revendication 7 visant à traiter la perte de mémoire.

18. Utilisation selon la revendication 7 visant à traiter la migraine.

19. Utilisation selon la revendication 7 visant à traiter la douleur.

20. Utilisation selon la revendication 19 dans laquelle le médicament est approprié pour une administration topique.
